# EUROPEAN PATENT SPECIFICATION

(11) **EP 0 648 720 B1**
(45) Date of publication and mention of the grant of the patent: **09.04.1997**
(21) Application number: 93870194.3
(22) Date of filing: 20.09.1993
(51) Int. Cl.: C07C 7/148

(54) **Process for the removal of arsine and carbonyl sulphide from hydrocarbon feedstocks containing light olefin(s)**
Verfahren zur Entfernung von Arsien und Carbonylsulfid aus leichten Olefinen enthaltender Kohlenwasserstoffbeschickungen
Procédé pour l'élimination d'arsine et de sulfure de carbonyle à partir de courants d'hydrocarbures contenant des oléfines légères

(43) Date of publication of application: 19.04.1995
(73) Proprietor: FINA RESEARCH S.A., B-7181 Seneffe (Feluy) (BE)
(72) Inventor: Bodart, Philippe, B-4480 Engis (BE); Belloir, Pierre, B-7090 Braine-le-comte (BE)

(56) References cited:
- EP-A- 0 308 569
- BE-A- 902 942
- GB-A- 2 242 199

## Description

### FIELD OF INVENTION

The present invention relates to a process for the consecutive removal of arsine and carbonyl sulphide (COS) from light olefin-containing hydrocarbon feedstocks, more particularly from propylene feedstocks.

### BACKGROUND OF THE INVENTION

Industrial applications of light olefin-containing hydrocarbons, and particularly liquified propylene, have become more increasingly specialised. The present technology uses highly efficient catalysts to convert these hydrocarbon feedstocks into final products such as polymers. However, those highly efficient catalysts are very sensitive to contaminants, particularly COS and arsine, found in said hydrocarbons.

The presence of COS or arsine, even at very low concentrations, oftentimes renders olefins, particularly propylene, valueless for many purposes. For example, high purity olefins are required for the satisfactory production of many polymeric products, especially those useful as plastics, including polymers of propylene. However, COS and arsine appear to be able to cause deactivation of the catalysts required for the polymerization . As a result, there is a real need to provide and improve techniques for removing COS and/or arsine from light olefin-containing hydrocarbons, particularly from those used for polymer production.

The purification of propylene is particularly complicated by the small difference between the boiling points of propylene, COS and arsine, which prevents the use of fractionation.

Belgian Patent 902 942 discloses a process for the removal of COS from liquid hydrocarbon feedstocks containing propylene and up to 70 ppm COS, by passing it through an absorbent consisting of nickel deposited on a support, the nickel being present both as metal and as oxide, the amount of nickel present as metal being comprised between 35 and 70 wt% of the total amount of nickel.

European Patent 448 698 discloses that the capacity of the absorbent disclosed in Belgian Patent 902 942 to remove COS from liquid hydrocarbons could be increased by a heat treatment between 150 and 450°C under a flow of non-oxidising gas.

European Patent 308 569 discloses a process for the removal of arsine from light olefin-containing hydrocarbon feedstocks, by passing it through an absorbent consisting of nickel deposited on a support, the nickel being present both as metal and as oxide, metallic nickel representing 10 to 50 wt.% of the absorbent.

### SUMMARY OF THE INVENTION

In accordance with the present invention, arsine and COS are removed consecutively by passing the hydrocarbon feed over an absorbent material comprising nickel deposited on a support material and wherein the nickel is present both as metallic nickel and as nickel oxide, the total weight of nickel and nickel oxide representing up to about 80% of the weight of the absorbent, the weight ratio of metallic nickel to nickel oxide being of from 0.4 to 2.0, with the proviso that metallic nickel should represent neither less than 10 wt% nor more than 50 wt% of the absorbent; said absorbent has been previously saturated with respectively COS or arsine.

### DETAILED DESCRIPTION

The present invention relates to the consecutive removal of COS and arsine, from light olefin-containing hydrocarbon streams. The present invention provides for a process for removing a first impurity selected from arsine and COS from a light olefin-containing hydrocarbon feedstock, said process comprising the steps of
a) passing said feedstock over an absorbent material comprising nickel deposited on a support material wherein said nickel is present as both nickel oxide and metallic nickel, said material having previously been saturated with a second different impurity selected from arsine and COS; and
b) recovering a hydrocarbon stream having a substantially reduced content in said first impurity.
Light olefins, as used herein, are C₂-C₆ olefins. Of particular interest is the treatment of hydrocarbon streams containing light olefins which are to be subsequently subjected to polymerization using polymerization catalysts. As stated previously, hydrocarbon streams containing propylene present a special problem because of the near boiling points of propylene, COS and arsine. While the subsequent discussion will describe the invention in terms of treating propylene-containing feeds, it should be understood that the present invention is applicable to the treatment of light olefin-containing hydrocarbon streams in general, i.e., hydrocarbon streams containing ethylene, propylene, butenes, pentenes, hexenes, or any combination thereof.

The process of the present invention is capable of reducing the COS and arsine concentrations in the treated hydrocarbon feedstock to 50 parts per billion by weight (ppb) or lower. The original COS and arsine concentrations are often as high as 1000 parts per million by weight (ppm), depending on the process of making and the origin of the hydrocarbon feedstock; due to the expense and specialization of the present invention, it is preferred to utilize other less costly and less complex processes to reduce the COS and arsine concentrations to 70 ppm or less prior to treatment with the absorbent of the present invention, to avoid excessive reductions of the space velocity.

The absorbent material of the present invention comprises nickel deposited on a support material, the nickel being present both as metallic nickel and as nickel oxide. Silica, silico-aluminas, alumina, kieselguhr, zeolites and other similar materials, whether amorphous or crystalline, can be utilized as the support. The total weight of nickel and nickel oxide may represent up to about 80 wt.% of the absorbent material, with the provision that metallic nickel should neither represent less than 10 wt.% nor more than 50 wt.% of the absorbent. Preferably, the weight ratio of metallic nickel to nickel oxide is of about 0.4 to about 2.0, and the absorbent comprises from about 30 to about 60 wt.% of support material. When carrying out the process of the invention with an absorbent material outside this definition, the results obtained may no longer be satisfactory, although some COS and some arsine will still be removed. Whilst not wishing to be bound by any theory, the Applicant believes that larger crystallites are formed if the Ni/NiO ratio is higher, thus leading to a lesser efficiency; similarly, an excessive total nickel content tends to lower the specific surface and consequently the efficiency, while a too low total nickel content would lead to an insufficient capacity for absorbing COS and arsine.

The nickel can be deposited on the support by any of the several methods well known to those skilled in the art. For example, nickel can be deposited on the support by dissolving nickel nitrate in water, mixing the solution with the support and precipitating the nickel, for example in the form of nickel carbonate, and subsequently washing, drying and calcining the precipitate. The nickel deposited in this manner in then partially reduced by means of hydrogen to form metallic nickel, the remainder being in the form of nickel oxide.

In general, the size of the nickel crystallites after reduction is from about 1 to about 2 nm. The size of the nickel crystallites depends on the extent of reduction carried out. In fact, if the degree of reduction is increased, the size of the crystallites is increased but the absorbent material obtained does not have the desired properties. On the other hand, if the degree of reduction is too low, the crystallites still have good dimensions but the quantity of nickel available in this case is too small to ensure successful purification of the feedstock.

The specific surface area of the absorbent material obtained after reduction should preferably be greater than 100 m²/g.

The particle size of the absorbent material depends especially on the pressure drop allowed in the reactor; it has been noted, however, that it is advantageous to use the absorbent material in finely divided form.

Preferably, the particle diameter of this material when spherical does not exceed about 3.5 mm and is most preferably from about 1 to about 2.5 mm; when cylindrical particles are used, they preferably have a diameter of from about 1 to about 2 mm and a length from about 3 to about 8 mm. Trilobes of similar size may also be used.

Whilst not wishing to be bound by any theory, the Applicant believes that COS and arsine can react with both the nickel and the nickel oxide, to form respectively nickel sulphide and arsenic metal (which either forms an NiAs alloy or is deposited on the support, and water), whilst (as said above) partial reduction is essential for obtaining appropriate crystallite size and thus the desired properties.

The absorbent material is usually prepared ex situ and stored either under a convenient saturated liquid hydrocarbon, like cyclohexane or dodecane, or under a non-oxidizing atmosphere, like N₂. It can also be protected (hereinafter "stabilised") by deposition of a carbon dioxide layer on the surface, said layer protecting the absorbent material from air, thus allowing easy handling; the carbon dioxide layer must be removed before use of the material, e.g. by nitrogen stripping at about 200°C.

It has been found that propylene adsorbs onto the fresh absorbent material when contacted with the feedstocks containing propylene during the COS and arsine removal from said feedstocks, and that the propylene adsorption reaction occurring during start-up is exothermic. Under certain conditions, and particularly when the propylene-free absorbent material used is directly contacted with pure propylene, the temperature rise may be very important, more particularly at the surface of the material of which the temperature may be much higher than that measured with a thermocouple, and it may thus damage the absorbent material. In addition the high temperatures result in the initialisation of undesired side-reactions, more particularly propylene dimerization and trimerization. The dimers are hexenes which copolymerize with propylene and break the regularity of the linear chain of isotactic polypropylene. As a result, the copolymer has a lower crystallinity than polypropylene, and thus a lower melting point; its mechanical resistance is also lower.

The Applicant has found that an excessive increase in the temperature of the absorbent material can be avoided by previously conditioning the material by passing over said material an inert gas flow containing a minor amount of at least one light olefin, preferably propylene in a concentration of from about 0.1 to 5 vol %. The inert gas, which should contain the least possible amount of oxygen, is usually nitrogen. It is preferable to begin the conditioning procedure by passing essentially pure inert gas. The conditioning step is preferably carried out at about atmospheric pressure, at or below ambient temperature. It is continued until the olefin concentration at the outlet equals that introduced. It is also possible to monitor the passage of an exotherm, shown by thermocouples introduced with the absorbent material.

It is known that, when the absorbent material is prepared ex situ and protected by a monolayer of carbon dioxide (believed to be sorbed on the nickel surface), the absorbent material must be pretreated prior to its conditioning by passing therethrough, at a temperature of from about 150 to about 350°C, preferably at about 250°C and preferably at about atmospheric pressure, a gazeous flow comprising first an inert gas (containing the least possible amount of oxygen) then preferably (to remove any oxygen possible sorbed on the absorbent despite all precautions) a mixture of inert gas and hydrogen containing an increasing concentration of hydrogen, before purging it free of hydrogen, e.g. with an inert gas flow at about 250°C.

In utilizing the latest generation of Ziegler-type catalysts in the production of polypropylene, it is essential that the propylene feedstock contains less than 50 ppb and preferably less than 30 ppb of COS of arsine. It has been unexpectedly found that by passing the propylene feedstock over an absorbent material as hereinbefore described, the feedstock obtained has both COS and arsine contents not exceeding 50 ppb. This result is unexpected due to the degree of purity obtained and due to the fact that this process can be carried out either in the presence or preferably in the absence of water. In addition, it has unexpectedly been found that neither the absorption of arsine nor that of COS is very significantly reducing the capacity of the material for absorbing COS or arsine.

In polypropylene production, the hydrocarbon feedstock generally comprises more than 75 wt.% propylene, more particularly from about 85 to about 99 wt.% propylene, and up to about 10 ppm COS and/or arsine. In one embodiment of the present invention, the propylene feedstock is passed over the absorbent material at a temperature of from about -10°C to about 80°C, preferably of from about 10°C to about 40°C, and under sufficient pressure to keep the medium in the liquid phase. The weight hourly space velocity (WHSV) utilized is from about 0.1 to about 25 kg/kg.h and preferably from about 1 to about 10 kg/kg.h.

In polyethylene production, the hydrocarbon feedstock generally comprises more than 80 wt.% of ethylene, more particularly from about 90 to about 99 wt.%, and up to about 10 ppm of COS and/or arsine. In another embodiment of the present invention, the ethylene feedstock is passed over the absorbent material at a temperature of from about -10°C to about 80°C, preferably from about 10°C to about 40°C, under a pressure of at least 1 MPa, and with a WHSV of from about 0.1 to about 25 kg/kg.h, preferably of from about 1 to about 10 kg/kg.h.

The examples which follow are given in order to provide a better illustration of the process of the present invention, but without thereby reducing its scope.

### Example 1 (preparation of the absorbent)

Nickel was deposited on silica-alumina as the support, the nickel being present in both the form of NiO and of metallic nickel, the weight ratio of metallic nickel to nickel oxide being of 0.668, and the total weight of Ni+NiO being 56.7% of the weight of the absorbent.

The absorbent material was finely divided to give an average particle size of about 1mm. The specific surface area of the absorbent was of 145 m²/g, while its bulk density was of 0.72.

### Example 2 (simultaneous removal) (comparative example)

A liquid feedstock of polymer grade propylene containing less than 5 ppm water was doped with 150 ppm arsine and 150 ppm COS.

The said feedstock was passed in the upflow mode through the absorbent material at a temperature of 25°C, under a pressure of 1.5 MPa (sufficient to keep the feedstock in the liquid phase), and at at LHSV of 10 L/L.h. After 24 hours, the COS and arsine contents were both below 50 ppb (detection limit).

### Example 3 (arsine then COS)

A liquid feedstock of polymer grade propylene containing less than 5 ppm water was doped with 150 ppm arsine, then passed through the absorbent material as in example 2. The effluent contained less than 30 ppb arsine. Breakthrough occurred after about 96 hours, when 100 ppb arsine were detected in the effluent; assuming that all arsine was absorbed during the first 96 hours, the capacity of the absorbent may be calculated to be of at least 53 g arsine/kg absorbent, i.e. about 5 wt % As.

A liquid feedstock of polymer grade propylene containing less than 5 ppm water was doped with 270 ppm COS, then passed through the absorbent material as in example 2. The effluent contained less than 30 ppb COS. Breakthrough occurred after about 999 hours, when 120 ppb COS were detected in the effluent; assuming that all COS was absorbed during the first 999 hours, the capacity of the absorbent was calculated to be of at least 10.8 g COS per 100 g catalyst (initial weight before arsine absorption).

### Example 4 (COS then arsine)

Example 3 was repeated, except that absorption of COS preceded absorption of arsine.

Breakthrough of COS occurred after 999 hours, when 90 ppb COS were detected in the effluent; a similar calculation gave a capacity of 12.5 g COS PER 100 g catalyst (initial weight).

Breakthrough of arsine occurred after about 84 hours, when 110 ppb arsine were detected in the effluent; the calculated capacity was of at least 46 g arsine per kg absorbent (initial weight before COS absorption), i.e. about 4.4 wt % As.

A comparison of examples 3 and 4 shows that more than 85% of the initial capacity is still available for arsine or COS when the absorbent has previously been saturated with respectively COS or arsine. This is a very surprising result, unexpected in view of the prior art.

### Example 5

An absorbent material as described in Example 1 was prepared, stabilised using carbon dioxide and stored during one month.

The absorbent material was pretreated by passing a gaseous flow thereron, at a temperature of 180°C and under atmospheric pressure, said gaseous flow being formed first of nitrogen during 14 hours, then a mixture of nitrogen and hydrogen during a further 24 hours, the hydrogen concentration therein being increased by about 5 vol % per hour up to more than 95 vol %. The absorbent material was purged free of hydrogen with a nitrogen flow then cooled under a flow of nitrogen.

The absorbent material was then conditioned. A nitrogen flow was passed during 4 hours over the absorbent material, under atmospheric pressure, at a temperature of 20°C, and with a gaseous hourly space velocity (GHSV) of 125 l/l.h. During a further 12 hours, the conditioning was continued under the same conditions with nitrogen containing 1 vol % propylene.

The purification procedures of Examples 2 to 4 were repeated with the conditioned material. Results similar to Examples 2 to 4 were obtained.

## Claims

1. A process for removing a first impurity selected from arsine and COS from a light olefin-containing hydrocarbon feedstock, said process comprising the steps of
a) passing said feedstock over an absorbent material comprising nickel deposited on a support material wherein said nickel is present as both nickel oxide and metallic nickel, said material having previously been saturated with a second different impurity selected from arsine and COS; and
b) recovering a hydrocarbon stream having a substantially reduced content in said first impurity.

2. The process of Claim 1 wherein the total weight of said nickel oxide and metallic nickel represent up to 80 wt.% of the absorbent material, the weight ratio of metallic nickel to nickel oxide being of from 0.4 to 2.0 with the provision that metallic nickel does not represent less than 10 wt.% nor more than 50 wt.% of the absorbent.

3. The process of Claim 2 wherein the absorbent comprises from 30 to 60 wt.% of support material.

4. The process of any one of Claims 1 to 3 wherein said absorbent material has a specific surface area of at least 100 m²/g.

5. The process of any one of Claims 1 to 4 wherein the feedstock comprises more than 75% by weight of propylene, preferably from 85 to 99 wt.% of propylene.

6. The process of Claim 5 carried out at a temperature of from -10°C to 80°C, at a sufficient pressure to retain the feedstock in liquid phase, and a WHSV of from 0.1 to 25 kg/kg.h.

7. The process of any one of Claims 1 to 4 wherein the feedstock comprises more than 80 wt.% of ethylene, preferably from 90 to 99 %.

8. The process of claims 7 carried out at a temperature of from -10°C to 80°C, at a pressure of at least 1 MPa, and at a WHSV of from 0.1 to 25 kg/kg.h.

9. The process of either one of Claims 6 or 8 carried out at a temperature of from 10°C to 40°C and at a WHSV of from 1 to 10 kg/kg.h.

10. The process of any one of Claims 1 to 9 wherein the absorbent material is previously conditioned by passing a flow of inert gas containing a minor amount of at least one light olefin.

11. The process of Claim 10 wherein said light olefin in said inert gas is propylene in a concentration of from 0.1 to 5 vol %.

12. The process of either one of Claims 10 or 11 wherein the absorbent material is pretreated prior to its conditioning by passing therethrough, at a temperature of 150 to 250°C and preferably under atmospheric pressure, a gaseous flow comprising first an inert gas, then a mixture of inert gas and hydrogen containing an increasing hydrogen concentration.

## Patentansprüche

1. Verfahren zur Entfernung einer ersten aus Arsin und COS ausgewählten Verunreinigung aus einem leichte Olefine enthaltenden Kohlenwasserstoff-Ausgangsmaterial, wobei das Verfahren die folgenden Schritte umfaßt:
a) Führen des Ausgangsmaterials über ein Absorptionsmaterial, das auf einem Trägermaterial abgeschiedenes Nickel umfaßt, wobei das Nickel sowohl als Nickeloxid als auch als metallisches Nickel vorliegt, und das Material vorher mit einer zweiten unterschiedlichen aus Arsin und COS ausgewählten Verunreinigung gesättigt worden ist; und
b) Wiedergewinnen eines Kohlenwasserstoffstroms mit einem wesentlich verringerten Gehalt der ersten Verunreinigung.

2. Verfahren nach Anspruch 1, wobei das Gesamtgewicht des Nickeloxids und des metallischen Nickels bis zu 80 Gew.-% des Absorptionsmaterials ausmacht und das Gewichtsverhältnis von metallischem Nickel zu Nickeloxid von 0,4 bis 2,0 beträgt, mit der Maßgabe, daß metallisches Nickel nicht weniger als 10 Gew.-% und nicht mehr als 50 Gew.-% des Absorptionsmittels ausmacht.

3. Verfahren nach Anspruch 2, wobei das Absorptionsmittel 30 bis 60 Gew.-% des Trägermaterials umfaßt.

4. Verfahren nach einem der Ansprüche 1 bis 3, wobei das Absorptionsmaterial eine spezifische Oberfläche von mindestens 100 m²/g aufweist.

5. Verfahren nach einem der Ansprüche 1 bis 4, wobei das Ausgangsmaterial mehr als 75 Gew.-% Propylen, vorzugsweise von 85 bis 99 Gew.-% Propylen, umfaßt.

6. Verfahren nach Anspruch 5, das bei einer Temperatur von - 10 °C bis 80 °C, bei einem dazu ausreichenden Druck, daß das Ausgangsmaterial in der flüssigen Phase gehalten wird, und bei einer WHSV von 0,1 bis 25 kg/kg·h durchgeführt wird.

7. Verfahren nach einem der Ansprüche 1 bis 4, wobei das Ausgangsmaterial mehr als 80 Gew.-%, vorzugsweise von 90 bis 99% Ethylen umfaßt.

8. Verfahren nach Anspruch 7, das bei einer Temperatur von - 10 °C bis 80 °C, bei einem Druck von mindestens 1 MPa und bei einer WHSV von 0,1 bis 25 kg/kg·h durchgeführt wird.

9. Verfahren nach einem der Ansprüche 6 oder 8, das bei einer Temperatur von 10 °C bis 40 °C und bei einer WHSV von 1 bis 10 kg/kg·h durchgeführt wird.

10. Verfahren nach einem der Ansprüche 1 bis 9, wobei das Absorptionsmaterial vorher durch Vorbeiführen eines Stroms eines inerten Gases konditioniert wird, das eine geringere Menge von mindestens einem leichten Olefin enthält.

11. Verfahren nach Anspruch 10, wobei das leichte Olefin in dem inerten Gas Propylen in einer Konzentration von 0,1 bis 5 Vol.-% ist.

12. Verfahren nach einem der Ansprüche 10 oder 11, wobei das Absorptionsmaterial vor seiner Konditionierung dadurch vorbehandelt wird, daß ein gasförmiger Strom, der zunächst ein inertes Gas, dann ein Gemisch aus einem inerten Gas und Wasserstoff, das eine zunehmende Wasserstoffkonzentration enthält, bei einer Temperatur von 150 bis 250 °C und vorzugsweise unter atmosphärischem Druck hindurchgeführt wird.

## Revendications

1. Un procédé pour enlever une première impureté choisie entre l'arsine et le COS d'un courant d'hydrocarbures contenant des oléfines légères, ce procédé comprenant les étapes de
a) faire passer ce courant sur une matière absorbante comprenant du nickel déposé sur une matière de support dans laquelle ce nickel est présent à la fois sous forme d'oxyde de nickel et de nickel métallique, cette matière ayant au préalable été saturée par une seconde impureté différente choisie entre l'arsine et le COS; et
b) récupérer un courant d'hydrocarbures ayant une teneur sensiblement réduite de cette première impureté.

2. Le procédé selon la revendication 1 dans lequel le poids total de cet oxyde de nickel et nickel métallique représente jusqu'à 80% en poids de la matière absorbante, le rapport pondéral de nickel métallique à oxyde de nickel étant depuis 0,4 à 2,0 avec la condition que le nickel métallique ne représente ni moins de 10% en poids ni plus de 50% en poids de la matière absorbante.

3. Le procédé selon la revendication 2 dans lequel l'absorbant comprend de 30 à 60% en poids de matière de support.

4. Le procédé selon l'une quelconque des revendications 1 à 3 dans lequel cette matière absorbante a une surface spécifique d'au moins 100 m²/g.

5. Le procédé selon l'une quelconque des revendications 1 à 4 dans lequel le courant comprend plus de 75% en poids de propylène, de préférence depuis 85 à 99% en poids de propylène.

6. Le procédé selon la revendication 5 mis en oeuvre à une température depuis -10°C à 80°C, à une pression suffisante pour maintenir le courant en phase liquide et une WHSV de 0,1 à 25 kg/kg.h.

7. Le procédé selon l'une quelconque des revendications 1 à 4 dans lequel le courant comprend plus de 80% en poids d'éthylène, de préférence depuis 90 à 99%.

8. Le procédé selon la revendication 7 mis en oeuvre à une température depuis -10°C à 80°C à une pression d'au moins 1 MPa, et une WHSV de 0,1 à 25 kg/kg.h.

9. Le procédé selon l'une quelconque des revendications 6 ou 8 mis en oeuvre à une température de depuis 10°C à 40°C et à une WHSV de 1 à 10 kg/kg.h.

10. Le procédé selon l'une quelconque des revendications 1 à 9 dans lequel la matière absorbante est au préalable conditionnée en faisant passer un courant de gaz inerte contenant une quantité minime d'au moins une oléfine légère.

11. Le procédé de la revendication 10 dans lequel cette oléfine légère dans ce gaz inerte est le propylène en une concentration de depuis 0,1 à 5% en volume.

12. Le procédé selon l'une quelconque des revendications 10 ou 11 dans lequel la matière absorbante est prétraitée avant d'être conditionnée en faisant passer au travers de celle-ci à une température de 150 à 250°C et de préférence sous pression atmosphérique, un courant gazeux comprenant d'abord un gaz inerte, ensuite un mélange de gaz inerte et d'hydrogène contenant une concentration croissante d'hydrogène.
